## Europäisches Patentamt

## European Patent Office

## Office européen des brevets

(19)

(11) Publication number: **0 129 609**

**B1**

(12)

# EUROPEAN PATENT SPECIFICATION

(45) Date of publication of patent specification: **22.04.87**

(51) Int. Cl.⁴: **C 07 D 473/12**, A 23 F 5/22

(21) Application number: **83106094.2**

(22) Date of filing: **22.06.83**

(54) Recovery of caffeine adsorbed to activated carbon.

(43) Date of publication of application:
**02.01.85 Bulletin 85/01**

(45) Publication of the grant of the patent:
**22.04.87 Bulletin 87/17**

(84) Designated Contracting States:
**AT BE CH FR IT LI NL**

(56) References cited:
**EP-A-0 042 295**
**EP-A-0 076 620**
**EP-A-0 078 088**
**DD-A- 78 586**

(73) Proprietor: **HAG GF AKTIENGESELLSCHAFT**
**Hagstrasse**
**D-2800 Bremen 1 (DE)**

(72) Inventor: **Vitzthum, Otto G., Dr.**
**Upper Borg 170**
**D-2800 Bremen (DE)**
Inventor: **Werkhoff, Peter, Dr.**
**Christian-Andersen-Weg 27**
**D-2805 Stuhr 4 (DE)**
Inventor: **Gehrig, Manfred, Dr.**
**Lug-ins-Land 10**
**D-8069 Wolnzach (DE)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

EP 0 129 609 B1

Courier Press, Leamington Spa, England.

## Description

### Technical Field

The invention relates to a process for recovering caffeine from loaded activated carbon which is characterized by treating the loaded activated carbon with formic acid.

### Background Art

For health reasons vegetable products — mainly coffee — of reduced caffeine content have gained increasing significance. Thus, quite a number of processes have been developed which, in various ways, try to reduce the caffeine concentration. Normally organic solvents have been used for extraction of the hydrolyzed coffee beans. The recovery of the solvent has been effected by distillation, for example, in the course of which the caffeine is obtained as by-product.

In order to avoid any contamination of the vegetable material by solvent residues, carbon dioxide which is unobjectionable under the health aspect has recently been used as extractant (German Patents Nos. 2,005,293 and 2,212,281). The carbon dioxide solvent in this process is freed from dissolved caffeine by means of activated carbon.

Prior to recycling of spent activated carbon, the adsorbed substances are normally subjected to pyrolysis whereafter the carbon is thermally reactivated. On account of the usefulness of caffeine, such a procedure is uneconomical.

Hence, it is not surprising that efforts are being made to recover the adsorbed caffeine. Measures for desorbing the caffeine must be selected in consideration of the fact that active carbon is a very good adsorbent, a circumstance that renders desorption difficult. Moreover, the use of any agents that are objectionable under the health aspect is to be avoided because the extraction is effected with carbon dioxide for the very purpose of avoiding such agents.

According to the teaching of German OS 2,544,116 the adsorbate is desorbed with supercritical gases, especially carbon dioxide. Thereafter the dissolved adsorbate must be removed from the dissolving gas.

According to U.S. Patent No. 4,298,736 caffeine adsorbed to activated carbon is desorbed with a food-grade liquid solvent which may be an organic acid or alcohol. Acetic acid, propionic acid and butyric acid are literally mentioned as organic acids. The process is to be carried out preferably at temperatures about 100°C and the solvents, i.e., the acids, should have a high boiling point.

A process is known from DD—A—78 586 for recovering caffeine from caffeine-containing carbon in which the activated carbon loaded with the caffeine is extracted with 5 to 15%, preferably 7 to 10%, aqueous acids, preferably with heating. There can be used as extraction agents water-soluble inorganic acids such as hydrohallic acids, sulfuric acid, phosphoric acid or water-soluble organic acids such as formic acid, acetic acid or oxalic acid.

A need exists for a process with which it is possible to recover the caffeine from the activated carbon in an effective manner.

### Disclosure of the Invention

With the present invention it has been surprisingly found that desorption with formic acid which has a boiling point 18°C lower than that of acetic acid, the solvent preferred in the U.S. patent, permits considerably better results despite shorter treating periods and lower treating temperatures and the use of small amounts of treating acid. This could not be expected from the general teaching given in the U.S. patent. The general teaching disclosed in said U.S. patent rather points toward higher boiling solvents.

The process temperature preferably is within a range between 90 and 100°C. The use of higher temperatures also calls for pressures higher than atmospheric because formic acid boils at about 100°C.

The treating period is preferably within a range of about 2 to 3 hours; an amount of formic acid 10 to 20 times that of activated carbon, based on the weight of the latter, is preferred. In the present process, undiluted formic acid or mixtures with up to 22% of water are used, although the use of undiluted formic acid is preferred.

The process can be carried out batch-wise or continuously. Preferably the process of the invention is carried out in battery operation, i.e., the loaded activated carbon is first contacted with formic acid which has already desorbed caffeine in preceding treating stages, and in the last treating stage of the battery the activated carbon already partially freed from caffeine is contacted with fresh formic acid which desorbes all the residual caffeine from the activated carbon. In this way, the caffeine-loaded formic acid is obtained at one end of the battery and activated carbon freed from caffeine is obtained at the other end. After removal of excess solvent by steaming and/or drying the activated carbon can be re-used. The adsorption activity of the activated carbon diminishes slightly with each recycle step. High temperature activation of the carbon is necessary only after repeated recycling.

Separation of the recovered caffeine from the formic acid takes place most simply by distillation of the formic acid.

In the following examples the below indicated mode of operation was adopted:

The formic acid was forced by means of a pump first through a heat exchanger and thereafter through the heated extraction tube. After having passed through a further heat exchanger the formic acid fractions were removed and analyzed.

The test containers consisted of V4A steel (1.4571 = AISI 316). Further, materials for containers resistant to boiling formic acid can be found, for example, in the tables of materials issued by Messrs. Dechema.

In all the tests, the temperature was 100°C.

## Example 1

50 g of activated carbon having a caffeine content of 10.9% by weight were treated with a flow of 1 kg formic acid for a period of 150 minutes. 91% of the adsorbed caffeine were found in the formic acid. The remainder was still on the activated carbon. The solution had a caffeine concentration of about 0.5%. After half the extraction period, when 0.5 kg of formic acid had been forced through the activated carbon, 78% of the caffeine had been desorbed.

This example permits an impressive comparison with example 1 of the above mentioned U.S. Patent No. 4,298,736. Thus, in the known process only 73% caffeine were desorbed and recovered after 2 hours of treatment at 117°C with 10 times the amount of acetic acid, based on the weight of activated carbon. On the other hand, the present example shows that with 10 times the amount of formic acid at a temperature of 100°C 78% of the caffeine can be desorbed and recovered already after a time of $1\frac{1}{4}$ hour. The higher yield attained under considerably more favourable process conditions with formic acid in lieu of acetic acid is extraordinarily surprising. The expert could not expect this from the general teaching of the U.S. patent. Rather was it probable that under comparable conditions poorer results would be achieved with formic acid.

## Example 2

Another 50 g of activated carbon having a caffeine content of 10.9% by weight were extracted with 1 kg formic acid containing 0.5% by weight of caffeine from the preceding example. In this run, further 64% of the adsorbed caffeine were extracted. The solution containing about 0.8% by weight of caffeine.

## Claims

1. A process for recovering caffeine from activated carbon which is loaded with caffeine by treating the loaded activated carbon with formic acid, characterized in that undiluted formic acid or mixtures of formic acid with up to 22% of water are used.

2. Process according to claim 1 characterized in that the treatment is carried out in battery operation.

## Patentansprüche

1. Verfahren zur Rückgewinnung von Coffein von mit Coffein beladener Aktivkohle durch Behandlung der beladenen Aktivkohle mit Ameisensäure, dadurch gekennzeichnet, daß unverdünnte Ameisensäure oder Mischungen von Ameisensäure mit bis zu 22% Wasser verwendet werden.

2. Verfahren nach Anspruch 1, dadurch gekennzeichnet, daß die Behandlung in einer Batterieschaltung vorgenommen wird.

## Revendications

1. Un procédé pour la récupération de caféine à partir de charbon activé chargé de caféine en traitant le charbon activé chargé par de l'acide formique, caractérisé en ce que l'on utilise de l'acide formique non dilué ou des mélanges d'acide formique avec jusqu'à 22% d'eau.

2. Un procédé selon la revendication 1, caractérisé en ce que l'on conduit le traitement avec un fonctionnement en batterie.